Europäisches Patentamt

European Patent Office

Office européen des brevets

(19)

(11)  **EP 0 603 154 B1**

(12)  # EUROPÄISCHE PATENTSCHRIFT

(45)  Veröffentlichungstag und Bekanntmachung des Hinweises auf die Patenterteilung:
**19.08.1998   Patentblatt 1998/34**

(51) Int Cl.⁶: **C12Q 1/00**, C12M 1/40
// C12Q1/54

(21)  Anmeldenummer: **93890240.0**

(22)  Anmeldetag: **13.12.1993**

(54)  **Amperometrische Enzymelektrode**

Amperometric enzyme electrode

Electrode enzymatique ampérométrique

(84)  Benannte Vertragsstaaten:
**DE GB**

(30)  Priorität: **15.12.1992  AT 2485/92**

(43)  Veröffentlichungstag der Anmeldung:
**22.06.1994   Patentblatt 1994/25**

(60)  Teilanmeldung: **96101100.4 / 0 711 837**

(73)  Patentinhaber: **AVL Medical Instruments AG
8207 Schaffhausen (CH)**

(72)  Erfinder:
  • **Offenbacher, Helmut Dr.
    A-8020 Graz (AT)**
  • **Marsoner, Hermann, Dipl.-Ing. Dr.
    A-8151 Steinberg (AT)**

(74)  Vertreter: **Krause, Walter, Dr. Dipl.-Ing. et al
Patentanwälte Babeluk - Krause,
Mariahilfer Gürtel 39/17
1150 Wien (AT)**

(56)  Entgegenhaltungen:
**EP-A- 0 247 850**

  • **DATABASE WPI Week 8434, Derwent Publications Ltd., London, GB; AN 84-210584 & JP-A-59 122 939 (MATSUSHITA ELEC IND KK) 16. Juli 1984**
  • **DATABASE WPI Week 8542, Derwent Publications Ltd., London, GB; AN 85-259794 & JP-A-60 173 452 (FUJI ELEC CORP R&D LTD) 6. September 1985**
  • **DATABASE WPI Week 9348, Derwent Publications Ltd., London, GB; AN 93-377863 & AT-D-196 792 (AVL GES VERBRENNUNGSKRAFT & MESSTECHNIK ET AL.) 15. Oktober 1993 & AT-B-397 661 27. Juni 1994**

Bemerkungen:
Teilanmeldung 96101100.4 eingereicht am 26/01/96.

**Beschreibung**

Die Erfindung betrifft eine amperometrische Enzymelektrode zur Messung der Konzentration eines Enzymsubstrates in einer Probe mit einem auf bzw. in einem elektrisch leitenden, porösen Elektrodenmaterial immobilisierten bzw. adsorbierten Enzym, wobei das Elektrodenmaterial einen redoxinaktiven Leiter mit einem leitfähigen Pigment und einem selbst nicht leitenden Bindemittel und eine darin fein verteilte katalytische Substanz aufweist.

Die Substratbestimmung durch amperometrischen Biosensoren bzw. Enzymelektroden auf Basis der Wasserstoffperoxidmessung mit immobilisierten Oxidoreduktasen ist Gegenstand einer großen Anzahl von Patenten und Publikationen und spielt in der biochemischen Sensorik eine nicht unerhebliche Rolle. Bei der Messung von Substraten wie z.B. Glucose in Körperflüssigkeiten (Blut, Serum, interstitielle Flüssigkeit und Harn) stellt sich das Problem der Interferenzen. So verfälschen viele redoxaktive Metabolite und Medikamente wie z.B. p-Acetamol, Ascorbat, Harnsäure u. a. die Substratbestimmung nach diesem Meßprinzip erheblich bzw. machen diese sogar unmöglich.

In der EP 0 247 850 A1 wird eine Unterdrückung dieser Interferenzen durch den Einsatz von Elektroden erzielt, bei denen fein verteilte Partikel der Platinmetalle auf der Oberfläche einer porösen Schicht bestehend aus Graphit und einem porösen polymeren Bindemittel aufgebracht sind. Durch diese Maßnahme wird die Zersetzungsspannung von $H_2O_2$, die ohne Verwendung von Katalysatoren bei $\geq$ 600 mV liegt, drastisch d.h. unter 400 mV reduziert. Da die Zersetzungsspannung für die redoxaktiven Störsubstanzen nur minimal reduziert wird, kommt es zu einer deutlichen Unterdrückung der Interferenzen.

Die Aufbringung der Platinpartikeln erfolgt in der Regel elektrochemisch durch Redox-Reaktionen bzw. kann durch Besputterung bewerkstelligt werden. Für die Herstellung von Elektroden nach dem Prinzip der Dickschichttechnik bedeuten diese Aufbringungsarten mitunter eine kostenintensive Prozeßmodifizierung.

Wasserstoffperoxid zerfällt autoxidativ in Wasser und Sauerstoff. Bei Raumtemperatur ist die Zerfallsgeschwindigkeit allerdings unmeßbar klein, kann jedoch beispielsweise durch Metalle der Platingruppe katalysiert werden.

Wie aus der EP 0 247 850 A1 hervorgeht, katalisieren die Platinmetalle den autoxidativen Zerfall von $H_2O_2$, wobei bei der für die $H_2O_2$-Bestimmung relevanten Reaktion (1) eine Verschiebung der Zersetzungsspannung $E_O$ von $\geq$ 0.6 V auf < 0.4 V erfolgt.

$$H_2O_2 \leftrightarrow 2H^+ + 2e^- + O_2 \qquad\qquad (1)$$

Durch die hohe Leitfähigkeit der Platinmetallpartikel sind bei diesem Elektrodentyp Katalysator und Ableitelektrode ident.

Weiters wird in der JP-A-59122939 (DERWENT Publikation AN 84-210 584) ein Verfahren zur enzymatischen Bestimmung von Substanzen in biologischen Proben, z. B. Glukose in Urin, mittels einer Enzymelektrode beschrieben. Zur Verhinderung störender Interferenzen, hervorgerufen durch Substanzen, wie Ascorbat oder Harnsäure, werden die Proben vor der Messung mit einem Oxidationsmittel, beispielsweise $MnO_2$ und $Fe_2O_3$, in Kontakt gebracht. Dabei wird auf einem Testträger immobilisiertes $MnO_2$ in die zu messende Probe getaucht, mittels Oxidation die Interferenten entfernt, und die im Testträger festgehaltene Probenflüssigkeit in eine Pufferlösung überführt, mit der in der Enzymelektrode die Analytkonzentration bestimmt wird. Es soll damit erreicht werden, daß an die eigentliche Enzymelektrode keine große Ansprüche an Interferenzfreiheit, Stabilität, Biokompabilität u. s. w. gestellt werden müssen.

Aufgabe der vorliegenden Erfindung ist es, ausgehend von eingangs beschriebenen Enzymelektroden eine amperometrische Enzymelektrode vorzuschlagen, welche kostengünstig und einfach herzustellen ist, und auch bei der Herstellung nach dem Prinzip der Dickschichttechnik keine Probleme aufwirft.

Diese Aufgabe wird erfindungsgemäß dadurch gelöst, daß die katalytische Substanz des Elektrodenmaterials ein stabiles Oxid oder Oxidhydrat eines Übergangselementes der vierten Periode des Periodensystems der Elemente ist, welches in den redoxinaktiven Leiter eingebettet ist. Überraschenderweise wurde entdeckt, daß die für den autoxidaven Zerfall relevanten Katalysatoren der genannten Oxide bzw. Oxidhydrate, welche im Vergleich zu den Edelmetallen schlechte Leiter bzw. Isolatoren sind, die Oxidation von $H_2O_2$ gemäß obiger Gleichung (1) ebenfalls beeinflussen, d. h. $E_o$ erniedrigen und somit auch zur Interferenzunterdrückung herangezogen werden können.

Als katalytische Substanz kann somit an Stelle eines Edelmetalls erfindungsgemäß zumindest ein Material aus der Gruppe $MnO_2$, $FeOOH$, $Fe_3O_4$, $Fe_2O_3$, $Cr_2O_3$ oder $V_2O_5$ verwendet werden.

Zwei beispielshafte Ausführungsvarianten der Erfindung sehen vor, daß das Elektrodenmaterial Partikel der katalytischen Substanz, vorzugsweise $MnO_2$, und Aktivkohlepartikel aufweist, oder daß das Elektrodenmaterial Aktivkohlepartikel aufweist, an deren Oberfläche die katalytische Substanz, vorzugsweise $MnO_2$, fein verteilt vorliegt, wobei die Partikel jeweils durch ein mit Graphitpulver versetztes Polymerbindemittel zu einem porösen Gefüge verbunden sind.

Durch innige Vermengung von z.B. Mangandioxid mit Graphit in einer im Bezug auf das Polymerbindemittel überfüllten (d.h. geringer Bindemittelanteil) Elektrodenmasse wird der an sich als Isolator wirkende Katalysator durch Kontaktieren der Oberfläche mit einer Unzahl von Graphitpartikelbrücken, beziehungsweise durch Aufbringung in feinverteilter Form auf Aktivkohle quasi leitend gemacht. Dabei soll erfindungsgemäß das Verhältnis der pulver- bzw. partikelförmigen Komponenten des Elektrodenmaterials zum Polymerbindemittel mindestens 2:1 betragen.

Durch das Überfüllen hat das Polymerbindemittel lediglich Partikelkittfunktion, das resultierende Elektrodenmaterial zeichnet sich durch eine hohe Porosität und somit durch eine große aktive Oberfläche aus.

Zur Herstellung des Elektrodenmaterials wird z.B. $MnO_2$-Pulver mit Graphit bzw. mit Aktivkohle und Graphit sowie mit einem Polymerbindemittel zu einer Paste verarbeitet, deren Film nach Abdunsten des Lösungsmittels eine mehr oder weniger poröse Struktur und somit eine große aktive Oberfläche besitzt. Ferner kann z.B., $MnO_2$ durch geeignete Redoxreaktionen auf Aktivkohle gefällt und das resultierende Pulver analog des reinen Manganoxids mit Graphit und Polymerbindemittel in eine siebdruckfähige Paste verarbeitet werden.

Die Herstellung einer erfindungsgemäßen Enzymelektrode wird unter der Verwendung von Braunstein (Mangan (IV)oxid) als Elektrokatalysator näher beschrieben. Die nachfolgend beschriebene Bestimmung von $H_2O_2$ neben den oben angeführten Störsubstanzen wurde bei physiologischen pH-Werten durchgeführt.

## BEISPIELE (Herstellung des Elektrodenmaterials)

1) 20 Gewichtsteile Mangan(IV)oxid gepulvert werden mit 100 Gewichtsteilen Graphitpaste (z.B. Elektrodag 4233 SS der Firma AchesonColloiden B.V. Scheenda/Niederlande) vermengt und in einer Kugelmühle zu einer homogenen Paste aufgeschlossen.

2) 10 Gewichtsteile Mangandioxid sowie 10 Teile Aktivkohle werden analog Beispiel 1 mit 100 Gewichtsteilen Graphitpaste vermischt.

3a) Fällung von Mangan(IV)oxid auf Aktivkohle: 10 g Aktivkohle werden in 100 ml 1n.NaOH suspendiert und kurzzeitig bis zum Sieden erhitzt. Nach dem Abkühlen wird die Suspension in eine Lösung bestehend aus 1 g $KMnO_2$, und 500 ml 1n.NaOH gegossen. Unter Rühren wird solange Natriumsulfit gelöst in Wasser zugesetzt, bis sich die Lösung entfärbt hat.

3b) Fällung von $MnO_2$ durch Synproportionierung gemäß Gleichung (2).

$$2\,MnO_4^{-} + 3Mn^{3+} + 4\,OH^{-} \rightarrow 5\,MnO_2 + 2\,H_2O \qquad (2)$$

10 g Aktivkohle werden in 200 ml 1n.NaOH suspendiert und kurzzeitig zum Sieden erhitzt. Nach Abkühlen wird die Suspension in eine Lösung bestehend aus 0.2 g $KMnO_4$ und 200 ml Wasser eingetragen und mit einer verdünnten Mangan(II)-Lösung solange versetzt, bis Entfärbung eintritt.

Die gemäß Beispiel 3a) und 3b) erzeugten $MnO_2$-Aktivkohle-Gemische werden abfiltriert, mit dest. Wasser gründlich gewaschen und bei 100°C getrocknet.

10 Teile dieser Fällungen werden mit je 50 Gewichtsteilen Graphitpaste verrührt und in einer Kugelmühle analog Beispiel 1) verarbeitet.

Elektrodenpräparationen

Die vorliegenden Elektrolytmaterialien werden jeweils in Form eines 1.5 x 8 mm bzw. 2 x 1 mm großen rechteckigen Feldes auf eine mit Silberleitbahnen bedruckte Plexiglasplatte via Siebdruckverfahren aufgebracht, und die blanken Oberflächen der Silberleitbahnen mit einem Isolierlack abgedeckt.

Zur weiteren Unterdrückung von Interferenzen eingangs genannter redoxaktiver Metabolite kann das Elektrodenmaterial der erfindungsgemäßen Enzymelektrode eine probenseitige Deckschicht oder eine in den Poren des Elektrodenmaterials vorliegende Sperrschicht aufweisen, welche aus einem polyionischen Polymer besteht.

Erfindungsgemäß ist vorgesehen, daß das polyionische Polymer aus polykationischen Polymeren, Polykationen bildenden Polymeren oder aus Polymeren besteht, welche sowohl positive als auch negative, kovalent gebundene Gruppen aufweisen, wobei beispielsweise das polykationische Polymer Aminogruppen sowie quarternäre Ammoniumgruppen trägt, oder das sowohl positive als auch negative Gruppen tragende Polymer ein Biopolymer, vorzugsweise ein Protein ist.

Zum Zwecke der Interferenzstudien wurden Elektrodenflächen, die aus der Paste nach Beispiel 1) gefertigt wurden,

EP 0 603 154 B1

beispielsweise mit polyionischen Polymeren wie Nafion, Albumin oder Polyethylenimin/Albumin im Verhältnis 2:1 beschichtet und die albuminhältigen Beschichtungen nachträglich mit Glutardialdehyd vernetzt.

Elektroden, die entsprechend Beispiel 3a) bzw. 3b) gefertigt wurden, wurden sowohl direkt als auch nach der Beschichtung mit perfluoriertem Nafion (Firma Aldrich-Chemie, Steinheim/BRD), mit 0.2 $\mu$l einer 5%igen Glucoseoxidase(GOD)Lösung beschichtet und durch Vernetzung im Glutardialdehyddampf (30 Minuten bei Raumtemperatur) immobilisiert.

Messungen:

Die Messung der einzelnen Parameter erfolgt potentiostatisch als Gegenelektrode (Kathode) fungiert eine Ag/AgCl-Elektrode.

Durch Variation der angelegten Spannung wurden die für die jeweilige Elektrode optimalen Bedingungen eruiert.

Als Referenzsysteme wurden Pt-Blech, Edelmetall-Aktivkohle-Graphitelektroden sowie eine reine Graphitelektrode vermessen.

Die Gold-Kohleelektrode wurde durch Mischen von Aktivkohle, welche 5% kolloidal verteiltes, durch einen Redoxprozeß aus Goldchlorid abgeschiedenes Gold enthält, analog Beispiel 1 hergestellt. Die Ruthenium-Kohleelektrode wurde analog präpariert, das Ruthenium wurde jedoch via Ruthenium 5% auf Aktivkohle in die Elektrodenmasse eingebracht. Platinisierter Kohlenstoff wurde gemäß GB 21 91 003 B (H.P. Benetto et al.) präpariert und in Form eines 1.5x8mm großen Elektrodenplättchens ausgetestet.

Weiters wurde das Elektrodenmaterial variiert, indem analog Beispiel 1 $MnO_2$ durch $Fe_2O_3$, $Fe_3O_4$, $FeOOH$, $Cr_2O_3$ sowie $V_2O_5$ ersetzt wurde.

Als Testmedien wurden $H_2O_2$ (1.0, 2.0 und 5.0 mM), Ascorbat, p-Acetamol, Urat (jeweils 1.0 mM) sowie Glucose (2.5, 5.0. 10.0 mM), alle gelöst in Standard A (ISE-Elektrolyt der Fa. AVL MEDICAL INSTRUMENTS, pH = 7.38 sowie physiologisch relevanter ionaler Hintergrund) verwendet; als Referenz wurde ebenfalls Standard A eingesetzt.

Gemessen wurde der Strom in Abhängigkeit von den einzelnen Parametern. In Tabelle I werden $MnO_2$-Elektroden den Edelmetallelektroden (Au/C, Ru/C, Pt-Blech sowie platinisierter Kohlenstoff) sowie einer reinen Graphitelektrode in puncto optimale Spannung, Änderung des Stromflusses I($\mu$A) bei Variation der Konzentration des redoxaktiven Analyten ($H_2O_2$) bzw. der Störer gegenübergestellt, sowie die $H_2O_2$-Konzentration angegeben, die den Stromfluß bei Vorliegen von 1mM interferierender Substanz entspricht. Bei dieser Messerie weisen die Elektroden keine polyionische Deckschicht auf.

4

TABELLE I

| Elektrodenmat. | I (µA) bei Wechsel Std.A-Analytlösung | | | | | $H_2O_2$-Konzentration entspricht 1 mM | | | |
|---|---|---|---|---|---|---|---|---|---|
| | $H_2O_2$ 1mM | $H_2O_2$ 5mM | Ascorbat 1mM | p Acetamol 1 mM | Urat 1mM | Ascorbat | p Acetamol | Urat | Arbeits-Spannung |
| $MnO_2$/C | | | | | | | | | |
| gemäß 1 | -10.0 | -42.5 | -7,0 | -2,0 | -3,0 | 0.7 | 0.2 | 0.3 | 350 |
| 2 | -11,0 | -47,0 | -7,5 | -1,9 | -2,2 | 0.68 | 0.17 | 0.2 | 350 |
| 3a | - 5.5 | -25,0 | -3.2 | -0.9 | -1.3 | 0.58 | 0.16 | 0.23 | 350 |
| 3b | - 6.2 | -29.0 | -4.0 | -1.3 | -2.0 | 0.64 | 0.20 | 0.32 | 350 |
| Au/C | -0.65 | -1.65 | -15.7 | -5.8 | -3.4 | 24.2 | 8.90 | 5.23 | 500 |
| Ru/C | -3.0 | -8.6 | -7.0 | -4.4 | -2.0 | 2.33 | 1.46 | 0.66 | 400-500 |
| Pt-Blech | -2,6 | -5.5 | -6.0 | -1.0 | -0.6 | 2.30 | 0.38 | 0.23 | 450-500 |
| platinisierter Kohlenstoff | -6.0 | -32 | -3.0 | 0.0 | -2,5 | 0.5 | 0 | 0.41 | 350 |
| Graphit | -0.1 | -0.25 | -12,0 | -10.0 | -4.0 | 121 | 100 | 40 | 600 |

EP 0 603 154 B1

Die Erfindung wird im folgenden anhand von Zeichnungen näher erläutert. Es zeigen:

| Fig. 1 | eine schematische Schnittdarstellung einer amperometrischen Enzymelektrode nach der Erfindung, |
|---|---|
| Fig. 2 und Fig. 3 | Details aus Fig. 1, |
| Fig. 4 | eine Anordnung von Enzymelektroden, |
| Fig. 5 | einen Schnitt gemäß Linie V-V in Fig. 4, sowie die |
| Fig. 6a, 6b, 7a bis 7c und 8 | Meßdiagramme mit erfindungsgemäßen Enzymelektroden. |

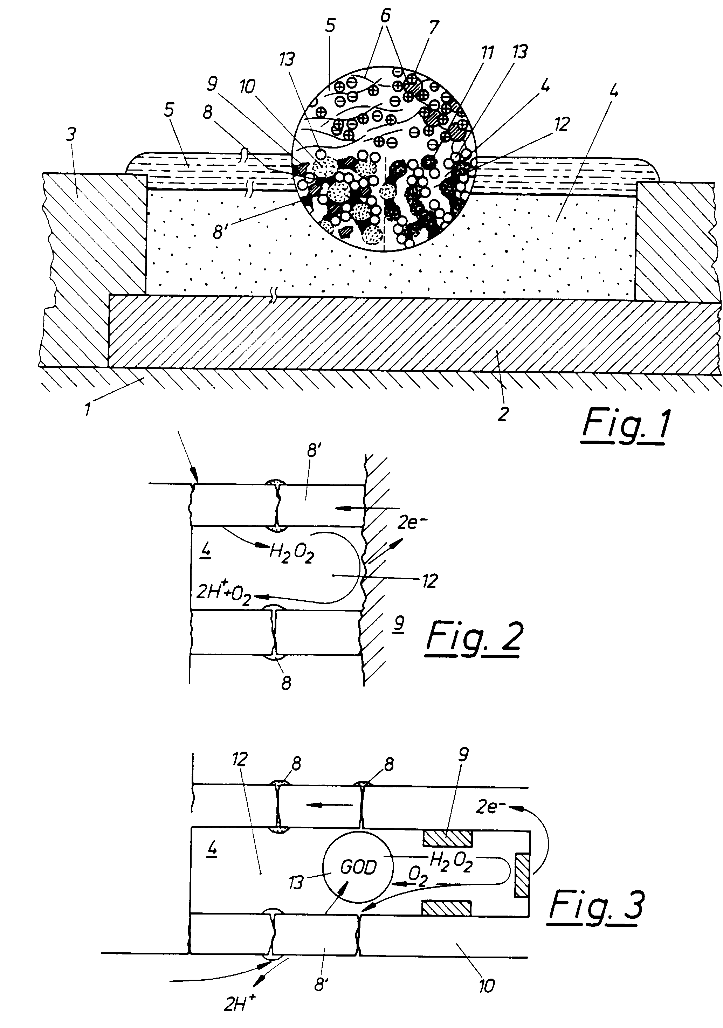

Gemäß Fig. 1 ist auf einem Träger 1 eine metallisch leitende Schicht 2, z.B. eine Silberleitbahn, aufgebracht, auf der sich ein elektrokatalytisches, poröses Elektrodenmaterial 4 befindet, sowie die darüber angeordnete permeationsselektive Deckschicht 5 angedeutet ist. Eine Isolierschicht 3 verhindert einen direkten Kontakt zwischen der Probe und der leitenden Schicht 2.

Das Elektrodenmaterial 4 weist ein poröses Gefüge auf und besteht aus Aktivkohlepartikel 10 und Partikel 9 einer katalytischen Substanz, beispielsweise Mangandioxidpartikel (linke Seite von Fig. 1) bzw. aus Aktivkohlepartikeln 11, an deren Oberfläche die katalytische Substanz feinst verteilt vorliegt (rechte Seite von Fig. 1). Die einzelnen Partikel 9, 10 und 11 werden durch ein Bindemittel 8 verkittet, in welchem sich als leitfähiges Pigment 8' Graphitpulver befindet. Die katalytische Substanz aus einem Oxid oder Oxidhydrat eines Übergangselementes der vierten Periode ist somit in einem redoxinaktiven Leiter aus einem Bindemittel 8 mit einem leitfähigen Pigment 8' eingebettet. In den Poren 12 des Elektrodenmaterials 4 ist das Enzym 13, beispielsweise Glucoseoxidase, adsorbtiv gebunden bzw. durch Vernetzung chemisch fixiert.

Die als permeationsselektive Interferenzsperrschicht fungierende Deckschicht 5 besteht aus einem linearen bzw. vernetzten globulären Polymergerüst 6, an dem positiv geladene Gruppen kovalent gebunden vorliegen. Aufgrund der Elektroneutralitätsbeziehung sind diese gebundenen Kationen mittels austauschbaren kleinen negativen Gegenionen (elektrostatisch fixierte Anionen 7') ladungskompensiert.

Die in den Poren 12 des porösen Elektrodenmaterials 4 ablaufenden chemischen bzw. elektrochemischen Reaktionen sind schematisch in den Fig. 2 und 3 dargestellt.

In Fig. 2 werden ein $MnO_2$-Partikel 9 über Graphitpartikel 8' kontaktiert, welche mit einem Polymerbindemittel 8 zusammengekittet sind. In Fig. 3 sind die $MnO_2$-Partikel 9 fein verteilt an die Aktivkohlepartikel 10 angelagert. Mit 13 ist das Enzym, beispielsweise Glucoseoxidase, gekennzeichnet.

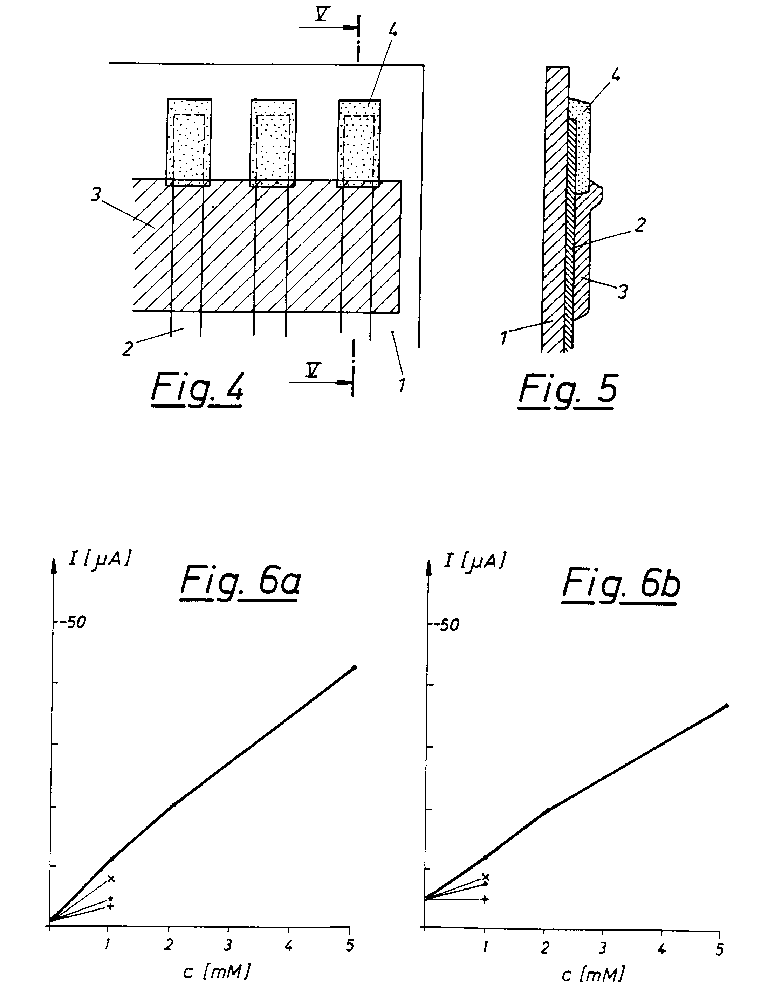

Die Fig. 4 und 5 zeigen eine Elektrodenanordnung, bei welcher auf einem Träger 1, beispielsweise einer Plexigasplatte mit Silberleitbahnen 2, das Elektrodenmaterial 4 in Form von 1,5 x 8 mm bzw. 2 x 1 mm großen rechteckigen Feldern aufgebracht wird. Die blanken Oberflächen der Silberleitbahnen können mit einer Isolierschicht 3 abgedeckt sein.

Tabelle II zeigt den Einfluß polyionischer Elektrodenbeschichtungen auf die Interferenzunterdrückung an einer $MnO_2$-Graphitelektrode.

TABELLE II

| $C_{H2O2}$ (mM) entspricht 1mM | | | | |
|---|---|---|---|---|
| ELEKTRODE | ASCORBAT | P ACETAMOL | URAT | OPTIMALE ARBEITS-SPANNUNG (mV) |
| $MnO_2$/C gemäß Beispiel 1 | 0.7 | 0.2 | 0.3 | 350 |
| +Nafion perfluoriert (Aldrich) | 0.09 | 0.07 | 0.21 | 340 - 350 |
| +Polyethylenimin/Albumin 2:1-m-Glutardialdehyd 3D-vernetzt | 0.21 | 0.08 | 0.07 | 350 |
| +Protein (Rinderserumalbumin mit Glutaldehyd 3 DI vernetzt | 0.1 | 0.15 | 0.05 | 350 |

Die Fig. 6a und 6b zeigen den Stromfluß I($\mu$A) versus Konzentration C(mM) der redoxaktiven Parameter (o = $H_2O_2$, x = p.Acetatamol, + = Ascorbat · = Urat) gemessen an einer $MnO_2$-Graphitelektrode (a) sowie an einer aus platinisiertem Kohlenstoff präparierten Elektrode (b).

In den Fig. 7a bis 7c ist der Stromfluß I($\mu$A) versus Konzentration C(mM) der redoxaktiven Parameter (gleiche Parameter wie Fig. 6a, 6b) gemessen an einer Mn02-Graphitelektrode (a und b) sowie an platinisierten Kohlenstoff (c) aufgetragen. Die Elektroden sind wie folgt modifiziert:

a = $MnO_2$/C-Nafion-GOD
b = $MnO_2$/C-GOD
c = platinisierter Kohlenstoff-Nafion-GOD

Fig. 8 zeigt den Stromfluß I($\mu$A) bei einer Spannung von 350 mV versus Glucosekonzentration bei einer optimierten erfindungsgemäßen Elektrode, bei der die permselektive Schicht auf die das Enzym beinhaltende Elektrodenschicht gemäß Fig. 1 aufgebracht ist. Die Funktion Strom versus Glucosekonzentration ist im Bereich von 0 bis 250 mg/dl (12,5 mM) linear.

Erfindungsgemäß kann das polyionisierte Polymer auch als Bindemittel für die pulver- bzw. partikelförmigen Elektrodenkomponenten fungieren.

Schließlich kann das polyionische Polymer das Enzym immobilisiert oder adsorbiert enthalten.

Die Messungen mit den erfindungsgemäßen Elektroden haben gezeigt, daß $MnO_2$-Graphit-Elektroden in puncto Verschiebung der Zersetzungspannung ein dem platinisierten Kohlenstoff analoges Verhalten zeigen, d.h. in Bezug auf Elektrokatalyse ebenbürtig sind. Erstaunlich ist, daß eine derartige starke Verschiebung weder bei Platinblech noch bei Ruthenium- bzw. Gold-Aktivkohle-Graphitelektroden beobachtet werden kann.

Bei den in der Tabelle I nicht ausgewiesenen Eisen-, Chromund Vanadiumoxid-Graphitelektroden (Herstellung analog Beispiel 1 der $MnO_2$-Kohleelektrode) konnte ein im Vergleich zu $MnO_2$ wesentlich schwächere Verschiebung der Zersetzungsspannung beobachtet werden, wobei folgende Reihenfolge auffiel: $MnO_2 > FeOOH > Fe_3O_4 > Fe_2O_3$-gebrannt $> Cr_2O_3, > Fe_2O_3$-mineralisch (Hämatit).

Wie aus der Tabelle I ebenfalls ersichtlich ist, nimmt mit der Verschiebung der Zersetzungsspannung auch die Interferenzunterdrückung zu.

Bezüglich der Aufbringung bzw. Einbringung polyionischer Polymere hat sich ergeben, daß die anionischen Interferenten (Ascorbat, Urat sowie der dissoziierte Anteil von p. Acetamol) z.B. durch Beziehen der Elektrode mit einer polyanionischen Deckschicht durch elektrostatische Abstoßung an der Wanderung zur Anode gehindert werden.

Aus Tabelle II ist ersichtlich, daß dieser Effekt nicht nur durch Polymere mit negativ geladenen Gruppen sondern auch durch überwiegend positiv geladene Polymerme aber auch durch generell polyionische Polymere erzielt werden kann. In diesem Fall scheint weniger die elektrostatische Barriere an der Grenzfläche zwischen Polymer und Probenmedium sondern vielmehr der Ionentauscheffekt solcher Polymere (Absenken der Diffusionsrate in Polymerfilm durch permanentes Ad- bzw. Desorbieren der Störionen beim Durchtritt) analog der Ionentausch-Chromatographie eine Rolle zu spielen. Im Falle von Proteinsperrschichten tritt somit bei den unter physiologischen Bedingungen vollständig dissoziierten Störern eine deutliche Interferenzunterdrückung auf.

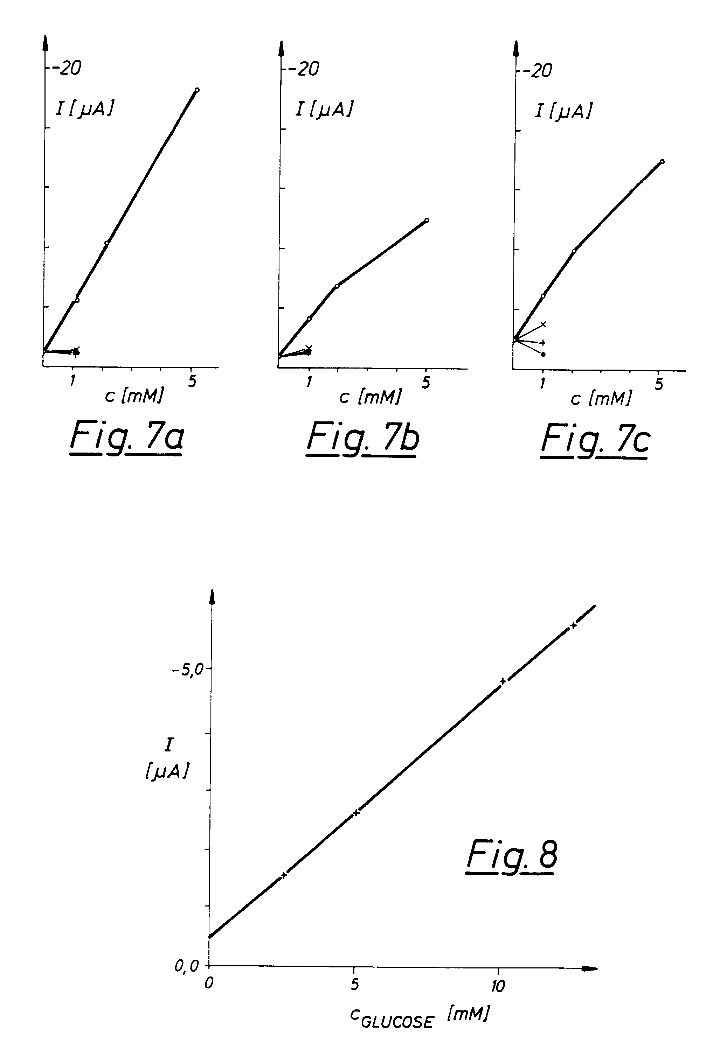

Tabelle III sowie die Fig. 7a-7c und 8 zeigen, daß im Bezug auf die $H_2O_2$ und Glucose-Messung bei der erfindungsgemäßen Elektrode im Vergleich zur Edelmetallkatalyse eine zum Teil bessere Interferenzunterdrückung gewährleistet ist.

TABELLE III

| ELEKTRODE | mM Glucose entspricht 1mM | | |
| --- | --- | --- | --- |
| | ASCORBAT | P ACETAMOL | URAT |
| $MnO_2$/C + Glucoseoxidase immobilisiert | + 1 | + 0.7 | + 0.5 |
| $MnO_2$/C + Nafion +Glucoseoxidase immobilisiert | - 0.2 | + 0.2 | 0 |
| Platinisierter Kohlenstoff + Nafion + Glucoseoxidase immobilisiert | 0 | + 5 | - 4 |

## Patentansprüche

1. Amperometrische Enzymelektrode zur Messung der Konzentration eines Enzymsubstrates in einer Probe mit einem auf bzw. in einem elektrisch leitenden, porösen Elektrodenmaterial (4) immobilisierten bzw. adsorbierten Enzym (13), wobei das Elektrodenmaterial (4) einen redoxinaktiven Leiter mit einem leitfähigen Pigment (8') und einem selbst nicht leitenden Bindemittel (8) und eine darin fein verteilte katalytische Substanz (9) aufweist, **dadurch gekennzeichnet,** daß die katalytische Substanz (9) des Elektrodenmaterials (4) ein stabiles Oxid oder Oxidhydrat eines Übergangselementes der vierten Periode des Periodensystems der Elemente ist, welches in den redoxinaktiven Leiter eingebettet ist.

2. Enzymelektrode nach Anspruch 1, **dadurch gekennzeichnet,** daß die katalytische Substanz (9) des Elektrodenmaterials (4) aus zumindest einem Material aus der Gruppe $MnO_2$, FeOOH, $Fe_3O_4$, $Fe_2O_3$, $Cr_2O_3$ oder $V_2O_5$ besteht.

**3.** Enzymelektrode nach Anspruch 1 oder 2, **dadurch gekennzeichnet,** daß das Elektrodenmaterial (4) Partikel (9) der katalytischen Substanz, vorzugsweise $MnO_2$, und Aktivkohlepartikel (10) aufweist, welche durch ein mit Graphitpulver (8') versetztes Polymerbindemittel (8) zu einem porösen Gefüge verbunden sind.

**4.** Enzymelektrode nach Anspruch 1 oder 2, **dadurch gekennzeichnet,** daß das Elektrodenmaterial (4) Aktivkohlepartikel (10) aufweist, an deren Oberfläche die katalytische Substanz (9), vorzugsweise $MnO_2$, fein verteilt vorliegt, wobei die Aktivkohlepartikel (10) durch ein mit Graphitpulver (8') versetztes Polymerbindemittel (8) zu einem porösen Gefüge verbunden sind.

**5.** Enzymelektrode nach einem der Ansprüche 1 bis 4, **dadurch gekennzeichnet,** daß das Verhältnis der pulver- bzw. partikelförmigen Komponenten (8', 9, 10) des Elektrodenmaterials (4) zum Polymerbindemittel (8) mindestens 2:1 beträgt.

**6.** Enzymelektrode nach einem der Ansprüche 1 bis 5, **dadurch gekennzeichnet,** daß das Elektrodenmaterial (4) eine probenseitige Deckschicht (5) oder eine in den Poren (12) des Elektrodenmaterials (4) vorliegende Sperrschicht aufweist, welche aus einem polyionischen Polymer besteht.

**7.** Enzymelektrode nach Anspruch 6, **dadurch gekennzeichnet,** daß das polyionische Polymer aus polykationischen Polymeren, Polykationen bildenden Polymeren oder aus Polymeren besteht, welche sowohl positive als auch negative, kovalent gebundene Gruppen aufweisen.

**8.** Enzymelektrode nach Anspruch 7, **dadurch gekennzeichnet,** daß das polykationische Polymer Aminogruppen sowie quarternäre Ammoniumgruppen trägt.

**9.** Enzymelektrode nach Anspruch 7, **dadurch gekennzeichnet,** daß das sowohl positive als auch negative Gruppen tragende Polymer ein Biopolymer, vorzugsweise ein Protein ist.

**10.** Enzymelektrode nach einem der Ansprüche 7 bis 9, **dadurch gekennzeichnet,** daß das polyionische Polymer als Bindemittel (8) für die pulver- bzw. partikelförmigen Elektrodenkomponenten (8', 9, 10) fungiert.

**11.** Enzymelektrode nach einem der Ansprüche 7 bis 10, **dadurch gekennzeichnet,** daß das polyionische Polymer das Enzym (13) immobilisiert oder adsorbiert enthält.

**Claims**

**1.** An amperometric enzyme electrode measuring the concentration of an enzyme substrate in a sample, with an enzyme (13) immobilized or adsorbed on or in an electrically conductive, porous electrode material (4) comprising a redox-inactive conductor with a conductive pigment (8'), and a non-conductive bonding agent (8), and a catalytic substance (9) dispersed therein, **characterized in that** said catalytic substance (9) of the electrode material (4) is a stable oxide or oxide hydrate of a transition element of the fourth period of the periodic system, which is embedded in the redox-inactive conductor.

**2.** An enzyme electrode as in claim 1, **characterized in that** said catalytic substance (9) of the electrode material (4) consists of at least one material from the group of $MnO_2$, $FeOOH$, $Fe_3O_4$, $Fe_2O_3$, $Cr_2O_3$, or $V_2O_5$.

**3.** An enzyme electrode as in claim 1 or 2, **characterized in that** the electrode material (4) contains particles (9) of the catalytic substance, preferably $MnO_2$, and particles (10) of activated carbon, which are bonded to form a porous structure by means of a polymer bonding agent (8) to which powdered graphite (8') has been added.

**4.** An enzyme electrode as in claim 1 or 2, **characterized in that** the electrode material (4) contains particles (10) of activated carbon whose surface is covered with the finely divided catalytic substance (9), preferably $MnO_2$, the carbon particles (10) being bonded to form a porous structure by means of a polymer bonding agent (8) to which powdered graphite (8') has been added.

**5.** An enzyme electrode as in any of claims 1 to 4, **characterized in that** the ratio between powdered or particle components (8', 9, 10) of the electrode material (4) and the polymer bonding agent (8) is at least 2:1.

6. An enzyme electrode as in any of claims 1 to 5, **characterized in that** the electrode material (4) is provided with a cover layer (5) on the side of the sample, or with a barrier layer sealing the pores (12) of the electrode material (4), which barrier consists of a polyionic polymer.

7. An enzyme electrode as in claim 6, **characterized in that** the polyionic polymer consists of polycationic polymers, polymers forming polycations, or polymers with both positive and negative groups that are covalently bonded.

8. An enzyme electrode as in claim 7, **characterized in that** the polycationic polymer carries amino groups and quaternary ammonium groups.

9. An enzyme electrode as in claim 7, **characterized in that** the polymer carrying both positive and negative groups is a biopolymer, preferably a protein.

10. An enzyme electrode as in any of claims 7 to 9, **characterized in that** the polyionic polymer acts as bonding agent (8) for the electrode components supplied as powders or particles (8', 9, 10).

11. An enzyme electrode as in any of claims 7 to 10, **characterized in that** the polyionic polymer contains the enzyme (13) in immobilized or adsorbed form.

## Revendications

1. Electrode ampérométrique à enzyme pour mesurer la concentration d'un substrat enzymatique dans un échantillon avec une sonde portant une enzyme (13) immobilisée ou absorbée, sur ou dans un matériau d'électrode poreux conducteur d'électricité (4), dans laquelle le matériau d'électrode (4) comprend un conducteur à activité redox avec un pigment conducteur (8'), un liant (8) intrinséquement non conducteur et une substance catalytique finement divisée (9), **caractérisée en ce que** la substance catalytique (9) du matériau d'électrode (4) contient un oxyde ou un oxyhydrate d'un élément de transition de la quatrième période du tableau périodique des éléments, qui est incorporé au conducteur à activité redox.

2. Electrode à enzyme selon la revendication 1, **caractérisée en ce que** la substance catalytique (9) du matériau d'électrode (4) contient au moins un matériau du groupe constitué par $MnO_2$, $FeOOH$, $Fe_3O_4$, $Fe_2O_3$, $Cr_2O_3$ ou $V_2O_5$.

3. Electrode à enzyme selon la revendication 1 ou la revendication 2, **caractérisée en ce que** le matériau (4) d'électrode comprend des particules de la substance catalytique, de préférence $MnO_2$ et des particules de charbon actif (10) qui sont liées à un support poreux par un polymère liant (8) chargé de graphite en poudre (8').

4. Electrode à enzyme selon la revendication 1 ou la revendication 2, **caractérisée en ce que** le matériau (4) d'électrode présente des particules de charbon actif (10) portant sur leur surface des particules de substance catalytique (9) finement divisée, en particulier de $MnO_2$, les particules de charbon actif (10) étant fixées à un support poreux par l'intermédiaire d'un polymère liant (8) chargé de poudre de graphite (8').

5. Electrode à enzyme selon l'une des revendications 1 à 4, **caractérisée en ce que** le rapport du composant pulvérulent ou particulaire (8', 9, 10) du matériau d'électrode (4) sur le milieu polymère liant (8) est d'au moins 2 : 1.

6. Electrode à enzyme selon l'une des revendications 1 à 5, **caractérisée en ce que** le matériau d'électrode (4) a une couche de revêtement (5) du côté de l'échantillon ou une couche d'isolement se trouvant dans les pores (12) du matériau d'électrode (4), constituée par un polymère polyionique.

7. Electrode à enzyme selon la revendications 6, **caractérisée en ce que** le polymère polyionique est constitué par un polymère polycationique, un polymère formant un polycation ou un polymère portant des groupes aussi bien positifs que négatifs, liés par covalence.

8. Electrode à enzyme selon la revendications 7, **caractérisée en ce que** le polymère cationique porte aussi bien des groupes amine que des groupes ammonium quaternaire.

9. Electrode à enzyme selon la revendications 7, **caractérisée en ce que** le polymère portant aussi bien des groupes positifs que des groupes négatifs est un biopolymère, de préférence une protéine.

10. Electrode à enzyme selon l'une des revendications 7 à 9, **caractérisée en ce que** le polymère polyionique joue le rôle de liant (8) pour les composants d'électrode pulvérulents ou particulaires (8', 9, 10).

11. Electrode à enzyme selon l'une des revendications 7 à 10, **caractérisée en ce que** le polymère polyionique retient l'enzyme (14) immobilisée ou absorbée.

Fig. 1

Fig. 2

Fig. 3

*Fig. 4*

*Fig. 5*

$I\ [\mu A]$

*Fig. 6a*

−50

$c\ [mM]$

$I\ [\mu A]$

*Fig. 6b*

−50

$c\ [mM]$

_Fig. 7a_    _Fig. 7b_    _Fig. 7c_

_Fig. 8_